Europäisches Patentamt

European Patent Office

Office européen des brevets

(11) Publication number: **0 271 793**
**A2**

# (12) EUROPEAN PATENT APPLICATION

(21) Application number: 87118037.8

(22) Date of filing: 05.12.87

(51) Int. Cl.4: **A61M 5/32**

(30) Priority: 17.12.86 IT 4164086

(43) Date of publication of application:
**22.06.88 Bulletin 88/25**

(84) Designated Contracting States:
**AT BE CH DE ES FR GB GR LI LU NL SE**

(71) Applicant: **Bidoia, Gianfranco**
**Via Bressanone 3/A**
**I-35100 Padova(IT)**

(72) Inventor: **Bidoia, Gianfranco**
**Via Bressanone 3/A**
**I-35100 Padova(IT)**

(74) Representative: **Modiano, Guido et al**
**MODIANO, JOSIF, PISANTY & STAUB**
**Modiano & Associati Via Meravigli, 16**
**I-20123 Milan(IT)**

(54) Container for used needles with device for removing the needles from the syringes.

(57) The container according to the invention is constituted by a box-like, for example parallelepipedal, body (1) which is closed by a practically non-removable cover (2), accommodating the device (3) for the removal of the needle from the syringe. The device (3) is composed of a rectangular opening (4) having its shorter side (7,8) slightly longer than the diameter of the conical tang of the needle which is removably associated with the tab of the syringe. The opening is practically closed by an elastic blade (5) which, besides preventing the accidental exit of needles from the container (1), also acts as a spring to engage the conical tang (18) of the needle (17) at a side (21) of the opening, allowing the removal of the needle from the syringe (16) by simply inclining the latter of a small angle.

Fig.5

EP 0 271 793 A2

# CONTAINER FOR USED NEEDLES WITH DEVICE FOR REMOVING THE NEEDLES FROM THE SYRINGES

The present invention relates to a container for used needles with a device for removing the needles from the syringes.

It is known that in the medical field current syringes for injections are almost exclusively of the disposable type, which are used only once and then intended to be destroyed.

These syringes are constituted by a body in plastic material associated with a metallic needle.

The most dangerous part of the syringe is constituted by the needle, since it not only can cause injury for anyone who should accidentally make contact therewith, but can constitute a dangerous vehicle of infections since after use it is contaminated by the blood of the patient.

For this reason, after injection, the needles are normally removed from the respective syringes and stored in a rigid container which cannot be perforated by their points, while the syringes are inserted in plastic bags which are subsequently sent, together with the container of the needles, to be destroyed.

Devices are known in which the needle of the syringe is inserted while it is still connected to said syringe, said devices including electric means generating an electric discharge which destroys the needle itself.

. Non-reusable box-like cardboard containers, adapted for the containment of small-size used materials, are also known.

These devices, beside being very expensive, are not practical, since in any case they require an electric supply which is not directly available on the nurse-trolley which passes through the patients' wards.

In the case of cardboard boxes, the same may get wet, easily break and perforate. In any case these containers are touched every time they are used, especially proximate to their openings, which are always contaminated by the previously introduced waste.

In view of these problems, the aim of the present invention is to provide a container for used needles provided with a device which allows the removal of the needles from the syringes without a direct and manual action of the nurse.

A consequent important object is to provide a container and a device which do not require, for their operation, any connection to power supplies, for example to electric supplies.

Still another object is to provide a container wherein means for the removal of the needle are provided which are directly operatable simply by holding the body of the syringe.

Still another object is to provide a container from which the even accidental exit of the needles after they have been introduced therein is not possible.

Not least object is to provide a container which is completely destroyable in an incinerator and that in any case has a modest cost and which, before being destroyed, cannot be perforated by the needles it contains.

The intended aim and objects are achieved by a container for used needles with a device for the removal of the needles from the syringes, as defined in the appended claims.

The characteristics and advantages of the invention will become apparent from the detailed description of a preferred embodiment, given only by way of non-limitative example and illustrated in the accompanying drawings, wherein:

figure 1 is a perspective view of the container;

figure 2 is a first embodiment of the needle removal device;

figure 3 is a perspective view of a second embodiment of the needle removal device;

figure 4 is a view, in partial cross section, of a syringe with the needle still associated, inserted in the removal device;

figure 5 is a partial cross section view showing the needle uncoupling moment.

With reference to the above described figures, the container 1, according to the invention, is of substantially box-like shape, made of a plastic material which can be destroyed in ordinary incinerators, possibly in the shape of a truncated pyramid so that many containers can be stacked in one another for transport reasons without occupying excessive bulk.

Said container 1 is upwardly closed by a cover 2, also in plastic material, peripherally restrained to the container 1 to safely prevent its accidental opening, even in case of a fall, and so that it is very difficult to open even with the use of various types of tool. It is necessary to associate the cover to the container in this manner since when the needles are introduced in the container they are dangerous to touch and handle.

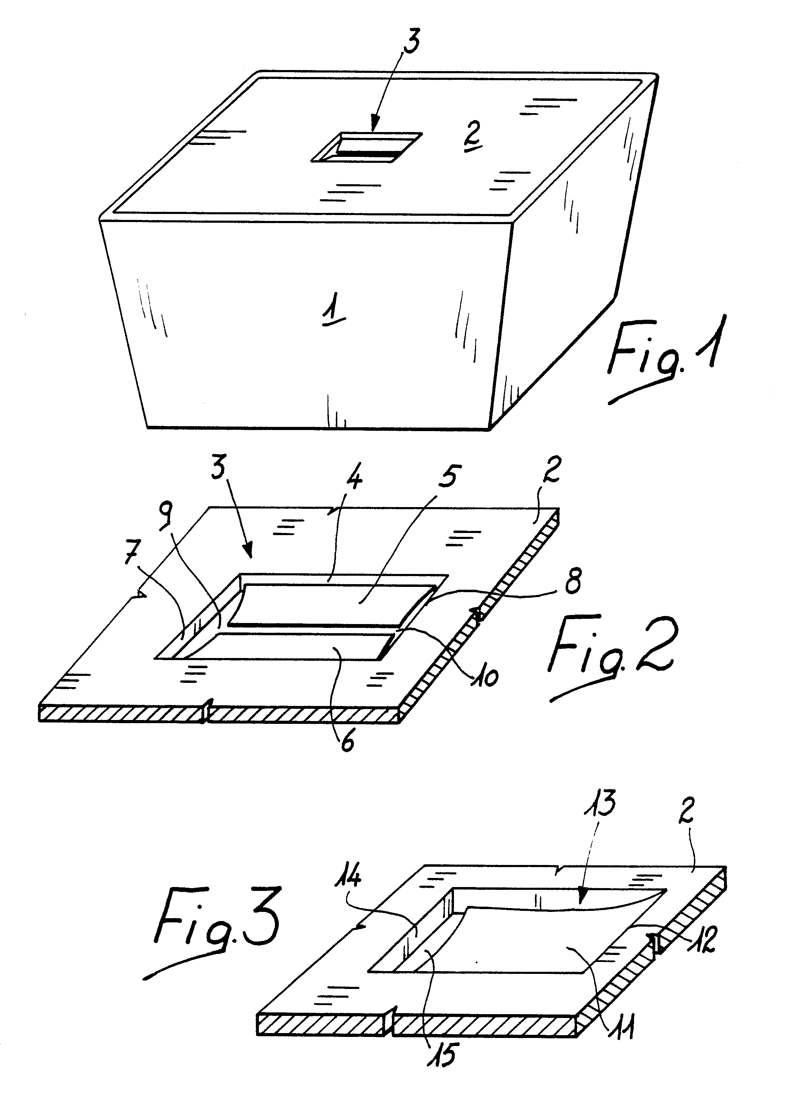

Preferably, the device for the removal of the needle from the syringe, generally indicated at 3, is provided in the middle central portion of the cover 2. It is illustrated in two different but equivalent embodiments in figures 2 and 3.

Substantially the device comprises a rectangular opening 4 which, in the case illustrated in fig. 2, is practically closed by two elastic blades, respectively 5 and 6, associated with the two long sides of the rectangular opening 4 and separated from the two short sides 7 and 8 of said opening 4.

The two blades 5 and 6 are elastically deformable and shaped so as to leave two small V-shaped openings indicated at 9 and 10 adjacent to the short sides 7 and 8 of the rectangular opening 4. In a second embodiment, illustrated in fig. 3, instead of two elastic blades 5,6, a single elastic blade 11 is provided associated with the side 12 of the rectangular opening, now indicated at 13 and which is spaced from the side 14, creating a passage gap 15. The two illustrated embodiments are substantially equivalent and in any case provide at least one elastic blade associated along one edge thereof with one of the sides of the opening provided in the cover 2 of the container 1.

Preferably, in both embodiments, the shorter side of the rectangular opening is slightly longer than the maximum diameter of the needle tang and shorter than the diameter of the syringe. Furthermore, the thickness of the container at the opening is smaller than the distance between the tang of the needle and the front part of the syringe.

The cover, as mentioned, is made of a plastic material and the elastic blades are provided in the same molding operation, being therefore rigidly associated with said cover.

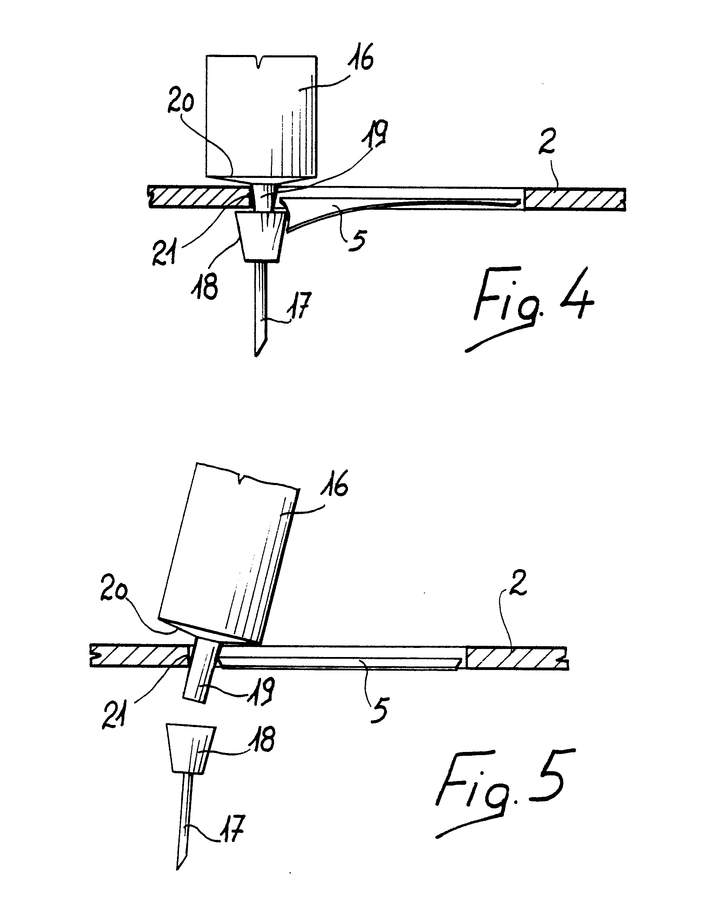

In order to perform the removal of the needle from the syringe, one proceeds as illustrated in figs. 4 and 5.

The syringe, indicated by 16, has the needle 17 associated by means of its conical tang 18 with the tab 19 protruding from the front part 20 of the syringe 16.

The needle is inserted in the rectangular opening 4 either at the space 9 or at the gap 15.

It can enter the container 1 by elastically deforming the blades, either 5 and 6 in the first embodiment or 10 in the second embodiment.

Once it fully enters the container, as an effect of the elastic thrust of the blades, the edge 21 of the cover 2 engages with the conical tang 18, while the latter is still associated with the syringe 16.

By now inclining the syringe as illustrated in fig. 5, the edge 21 acts on the tang 18, removing it from the tab 19 and causing it to fall into the container.

The syringe can now be removed to be stored in a second container which can be more simply a bag in plastic material.

The needle which has fallen into the container can no longer come out since the elastic blades, in the absence of the inserted syringe, practically close the opening 4.

From what has been described and illustrated it is apparent that a simple container for needles has been provided and that said container is provided with a device for removing the needle from the syringe which can be activated using only one hand, the one which holds the syringe, and without having in any case to touch the needle.

The operation is particularly safe and no type of accident is foreseeable which may cause the contact of the needle with the person holding the syringe.

In the removal operation no lateral thrusts are exerted on the container and therefore it cannot slide, nor are external power sources required for its operation.

It is thus apparent that all the intended aims have been achieved, providing a container which is particularly safe and easy to use.

Naturally, starting from the same inventive concept, other practical embodiments may be chosen, for example with an elastic blade connected to the opening along two adjacent sides thereof.

The materials may in any case be chosen always taking into account the fact that the container, which will have a daily-type use, is to be subsequently destroyed together with its content without producing, in incineration, toxic or harmful byproducts.

## Claims

1. A container (1) for used needles having a substantially box-like shape, characterized by a device(3) for the removal of the needle from the syringe provided on the upper face (2) of said container and comprising an opening (4;13), said opening being practically closed by at least one elastic blade (5,6;11) substantially countershaped to said opening and associated along at least one edge thereof with a side of said opening, said blade further having at least one free edge cooperating with said opening for engaging with the tang (18) of the needle (17).

2. A container according to claim 1, characterized in that it is made of a plastic material which cannot be perforated by the needles of the syringes and is completely destroyable by incineration.

3. A container according to claim 1, characterized in that said opening (4;13) is substantially rectangular and has its shorter side (7,8;14) which is slightly longer than the maximum diameter of the tang (18) of the needle (17) and shorter than the diameter of the syringe (16).

4. A container for needles according to any of the preceding claims, characterized in that said elastic blade (5,6;11) has rectangular shape connected at only one edge thereof with a corresponding side (12) of said opening (14;13)

5. A container according to any of the preceding claims, characterized in that said elastic blade (5,6;11) is shaped so as to form, with a free edge thereof, a small guiding aperture (9,10;15) in said opening (4;13) for the insertion of the needle (17).

6. A container according to claim 1, characterized in that the thickness of the container (1) at the opening (4;13) is smaller than the distance between the tang (18) of the needle (17) and the front part (20) of the syringe (16) when the needle is inserted on the syringe for normal use.

7. A container according to any of the preceding claims, characterized in that the inclination of the syringe (16) which determines the removal of the needle is equal to a few degrees from the vertical.

8. A container according to any of the preceding claims, characterized by two rectangular blades (5,6) having each a width which is substantially equal to half of the width of said opening (4), said blades being connected each, at a respective longer edge thereof, with a different longer side of said opening.

9. A container according to any of the preceding claims, characterized in that said opening (4;13) is formed in a cover (2) fixedly connected to said box-like container (1).

0 271 793

Fig.1

Fig.2

Fig.3

0 271 793

Fig. 4

Fig. 5